# FASCICULE DE BREVET EUROPEEN

(11) **EP 0 708 752 B1**
(45) Date de publication et mention de la délivrance du brevet: **22.04.1998**
(21) Numéro de dépôt: 94918698.5
(22) Date de dépôt: 11.07.1994
(51) Int. Cl.: C07C 53/126, C07C 51/41

(54) **PROCEDE DE PREPARATION DE DERIVES MAGNESIENS**
VERFAHREN ZUR HERSTELLUNG VON MAGNESIUMVERBINDINGEN
MAGNESIUM DERIVATIVE PREPARATION PROCESS

(30) Priorité: 16.07.1993 BE 9300748
(43) Date de publication de la demande: 01.05.1996
(73) Titulaire: LHOIST RECHERCHE ET DEVELOPPEMENT S.A., B-1342 Ottignies-Louvain-La-Neuve (BE)
(72) Inventeur: GOMBART, Marc, F-62910 Eperlecques (FR)
(74) Mandataire: Powis de Tenbossche, Roland
(86) Numéro de dépôt international: BE9400043
(87) Numéro de publication internationale: WO9502571

(56) Documents cités:
- FR-A- 2 261 335
- PATENT ABSTRACTS OF JAPAN vol. 9, no. 17 (C-262) (1740) 24 Janvier 1985 & JP,A,59 167 535 (NIHON KAGAKU SANGYO K.K.) 21 Septembre 1984
- PATENT ABSTRACTS OF JAPAN vol. 13, no. 72 (C-570) (3420) 17 Février 1989 & JP,A,63 264 547 (ASAHI DENKA KOGYO K.K.) 1 Novembre 1988

## Description

La présence invention a pour objet un procédé de préparation de mono ou dialkanoate en C₅⁺ de magnésium et en particulier de dialkanoate de magnésium.

Il est connu de réparer du stéarate de magnésium par préparation d'un milieu semi fondu d'acide stéarique (± 33% en phase liquide et ± 66% en phase solide), par ajout audit milieu d'un lait de magnésie obtenu à partir de MgO de faible réactivité (réactivité supérieure à 25 secondes calculée selon la méthode qui sera décrite plus loin) et par ajoût audit milieu d'un agent de réactivité tel que de l'acide formique pour démarrer la réaction. La réaction qui est faite dans une cuve (en batch) nécessite ± 30 minutes pour être sensiblement complète.

La teneur en humidité du produit de la réaction est supérieure à 8%, tandis que la teneur en acide gras est de l'ordre de 2%. Le produit ainsi obtenu, ensuite séché pendant ± 90 minutes et les blocs de produit séché sont enfin soumis à un broyage énergétique pour obtenir du stéarate de magnésium sous forme de poudre.

Ce procédé connu nécessite pour obtenir du stéarate de magnésium en poudre des étapes de séchage et de broyage coûteuses. De plus, ce procédé connu ne permet pas une fabrication en continu de stéarate de magnésium.

On connaît également par le document JP-A-59/167535 une réaction en solution aqueuse d'un oxyde, hydroxyde ou carbonate d'un métal choisi parmi le Zn, Ca, Mg, Zr, Co, Ni, Cu, Mn avec un acide gras à une température supérieure à la température de fusion du sel ainsi préparé. En fin de réaction, de l'eau froide est ajoutée.

La réaction de carbonate de magnésium avec un acide gras fondu est lente, voire impossible. Il en est de même si l'oxyde ou l'hydroxyde de magnésium ne présente pas une réactivité déterminée. A cet égard, des essais (voir tableau 2) montre que l'utilisation de MgO avec une réactivité de 30 secondes, réactivité qui est déjà importante, ne permet pas d'obtenir une conversion suffisante.

Le demandeur a utilisé lors d'essais, des oxydes de magnésium présentant une réactivité plus importante, (réactivité inférieure à 20 secondes) dans ce procédé connu. Les résultats de ces essais étaient négatifs puisqu'il n'a pas été possible d'obtenir une réaction sensiblement complète après plus de 20 minutes de réaction.

On connaît enfin par DD-86825 un procédé de préparation de stéarate de magnésium dans lequel on mélange, sous vide et sans apport d'énergie, 5,56 kg d'acide stéarique et 500g d'oxyde de magnésium.

La température du mélange atteignait 50°C, après 12 minutes et 73°C après 18 minutes. La réaction était terminée après 20 minutes. Le produit de réaction était un stéarate de magnésium avec une teneur en oxyde de magnésium de 7 à 8%, une teneur en acide stéarique de 1,35% et une teneur en eau de l'ordre de 0,2%.

Ce procédé ne permet pas la préparation de stéarate de magnésium conforme à la pharmacopée puisque le taux de conversion de l'oxyde de magnésium n'est que d'environ 90%, c'est-à-dire que la réaction est incomplète.

Ce procédé présente de plus l'inconvénient de ne pas pouvoir être effectué en continu puisqu'il y a lieu de mélanger intimement et de la façon la plus homogène possible des particules solides d'acide stéarique et des particules solides d'oxyde de magnésium.

La présente invention a pour objet un procédé de préparation de mono ou dialkanoate, à plus de 5 atomes de carbone, de magnésium dans lequel on fait réagir de l'oxyde et/ou de l'hydroxyde de magnésium avec un ou plusieurs acides carboxyliques possédant plus de 5 atomes de carbone en présence d'eau, procédé permettant une conversion d'au moins 95 %, de préférence d'au moins 97 %, du MgO et/ou Mg(OH)₂ en alkanoate de magnésium, après un temps de réaction de moins de 5 minutes, de préférence d'environ 3 minutes.

De plus, la réaction du procédé selon l'invention peut, si requis, être effectuée en continu, ceci réduisant en conséquence les investissements de l'installation de fabrication et ceci facilitant le contrôle de la réaction.

Dans le procédé suivant l'invention, on prépare dans une première étape un mélange d'acide(s) carboxylique et d'oxyde et/ou d'hydroxyde de magnésium porté à une température telle qu'au moins 80 % de l'acide soit sous forme fondue, l'oxyde et/ou hydroxyde de magnésium dudit mélange présentant une réactivité inférieure ou égale à 20 secondes selon la méthode qui sera décrite plus loin, et on ajoute, dans une deuxième étape, audit mélange de l'eau.

Le demandeur a remarqué qu'un ou des acide(s) carboxylique à plus de 5 atomes de carbone dont plus de 80 % est sous forme fondue et de l'oxyde et/ou de l'hydroxyde de magnésium ne réagissent pas si l'oxyde et/ou l'hydroxyde de magnésium présentait une haute réactivité, réactivité inférieure à 20 secondes et en absence d'eau.

De façon avantageuse, on utilise dans le procédé suivant l'invention de l'oxyde de magnésium présentant une réactivité inférieure à 16 secondes, par exemple comprise entre 10 et 15 secondes.

Un tel oxyde de magnésium peut être obtenu par cuisson de carbonate de magnésium à basse température, par exemple à une température de 600 à 700°C.

De façon avantageuse, on porte lors de la première étape, le mélange d'acide(s) carboxylique(s) et d'oxyde et/ou d'hydroxyde de magnésium, à une température telle que sensiblement tout l'acide ou tous les acides carboxylique(s) soient fondus.

Dans une forme de réalisation avantageuse, dans la deuxième étape, on ajoute une quantité d'eau telle que la teneur en eau du mélange au début de la réaction est au moins de 3%. De façon avantageuse, la quantité d'eau ajoutée est telle que la teneur en eau du mélange au début de la réaction est de 5 à 15%.

Selon une particularité d'une forme de réalisation, dans la première étape on soumet le mélange à une agitation de manière à avoir un mélange sensiblement homogène.

Dans une forme de réalisation, on fait réagir une quantité d'oxyde et/ou d'hydroxyde de magnésium avec de l'acide ou des acides carboxylique en C₅⁺ telle que le rapport molaire oxyde et/ou hydroxyde de magnésium/acide ou acides carboxyliques en C₅⁺ soit supérieur à 0,5 en début de réaction.

Dans une autre forme de réalisation, on fait réagir de l'oxyde de magnésium et/ou de l'hydroxyde de magnésium avec de l'acide ou des acides carboxyliques en C₅⁺ en présence d'une quantité d'eau telle qu'en fin de réaction, le produit de réaction contient moins de 10% en eau, de préférence moins de 2%, de sorte que le produit de réaction peut ne pas subir de séchage avant d'être broyé. De plus, le produit de réaction se trouve sous forme de petits agglomérats aisément broyables.

Dans des formes de réalisation particulières, dans la deuxième étape du procédé suivant l'invention, on ajoute au mélange de l'eau et un agent de réactivité (acide formique, acide maléique, acide acétique ou un mélange de ceux-ci).

D'autres particularités et détails de l'invention ressortiront de la description détaillée suivante de procédés de préparation de mono ou dialkanoate de magnésium donnés à titre d'exemples uniquement.

### A. Mesure de la réactivité de l'oxyde de magnésium

La réactivité de l'oxyde de magnésium a été mesurée de la manière suivante :

On a préparé une solution d'acide citrique 0,4N en dissolvant 141g d'acide citrique et 1,25g de benzoate de sodium dans de l'eau déminéralisée, en ajoutant 10 ml de phénolphtaleine et en complétant le mélange avec de l'eau déminéralisée pour obtenir 5000 ml. La solution ainsi obtenue est ensuite titrée avec du NaOH.

On a prélevé 50 ml de cette solution, on a porté cette solution à 30°C et on a ajouté à cette solution ig d'oxyde de magnésium. Cinq secondes après l'ajout de l'oxyde de magnésium, on a soumis le mélange à une agitation.

La réactivité de l'oxyde de magnésium est mesurée par le temps s'écoulant entre l'ajout de l'oxyde à la solution et le virage au rose de la solution (indicateur).

### B. Exemples de préparation de stéarate de magnésium

### Exemple n° 1

Dans cet exemple on a utilisé un oxyde de magnésium présentant une réactivité de 15 secondes. On a mélangé 35 g dudit oxyde de magnésium à 350 g d'acide stéarique fondu. La température du mélange était de 60°C. Aucune réaction n'a été observée dans ledit mélange tant que de l'eau n'était pas ajoutée au mélange.

La quantité d'eau ajoutée au mélange était de 30 g.

Dès que l'eau était ajoutée, la réaction exothermique de formation de stéarate de magnésium débutait. Après trois minutes, la réaction était terminée. Le stéarate de magnésium ainsi préparé avait une teneur en acides gras libres inférieure à 0,5% et une teneur en humidité de l'ordre de 0,3%.

### Exemples 2 à 7

Dans ces exemples, on a préparé du stéarate de magnésium de la manière décrite dans l'exemple 1 si ce n'est qu'on a fait varier la température de réaction (T) et/ou la quantité d'eau ajoutée (Z).

La tableau suivant donne pour ces exemples le temps de réaction (t), la teneur en acides gras libres en % (%AGL) et la teneur en humidité (%H₂O) du stéarate de magnésium préparé.

**TABLEAU 1**

| Exemple | 2 | 3 | 4 | 5 | 6 | 7 |
|---|---|---|---|---|---|---|
| T (°C) | 60 | 65 | 65 | 70 | 75 | 80 |
| Z(g) | 40 | 30 | 40 | 30 | 30 | 30 |
| % AGL | < 0,5 | < 0,5 | < 0,5 | < 0,5 | <0,5 | <0,5 |
| % H₂O | 0,2 | 0,3 | 0,2 | 0,3 | 0,2 | 0,3 |
| t (s) | 120 | 170 | 110 | 150 | 140 | 120 |

### Exemples 8 à 18

Dans ces exemples, on a préparé du stéarate de magnésium de la manière décrite dans l'exemple 1, si ce n'est qu'on a utilisé un oxyde de magnésium de réactivité (R) différente et qu'on a fait varier la température de la réaction (T).

Le tableau suivant donne pour ces exemples de préparation, la réactivité (en secondes) de l'oxyde de magnésium utilisé, la température de la réaction, la teneur en acides gras libres (% AGL) et la teneur en humidité (% H₂O) du stéarate de magnésium préparé et enfin le temps de réaction.

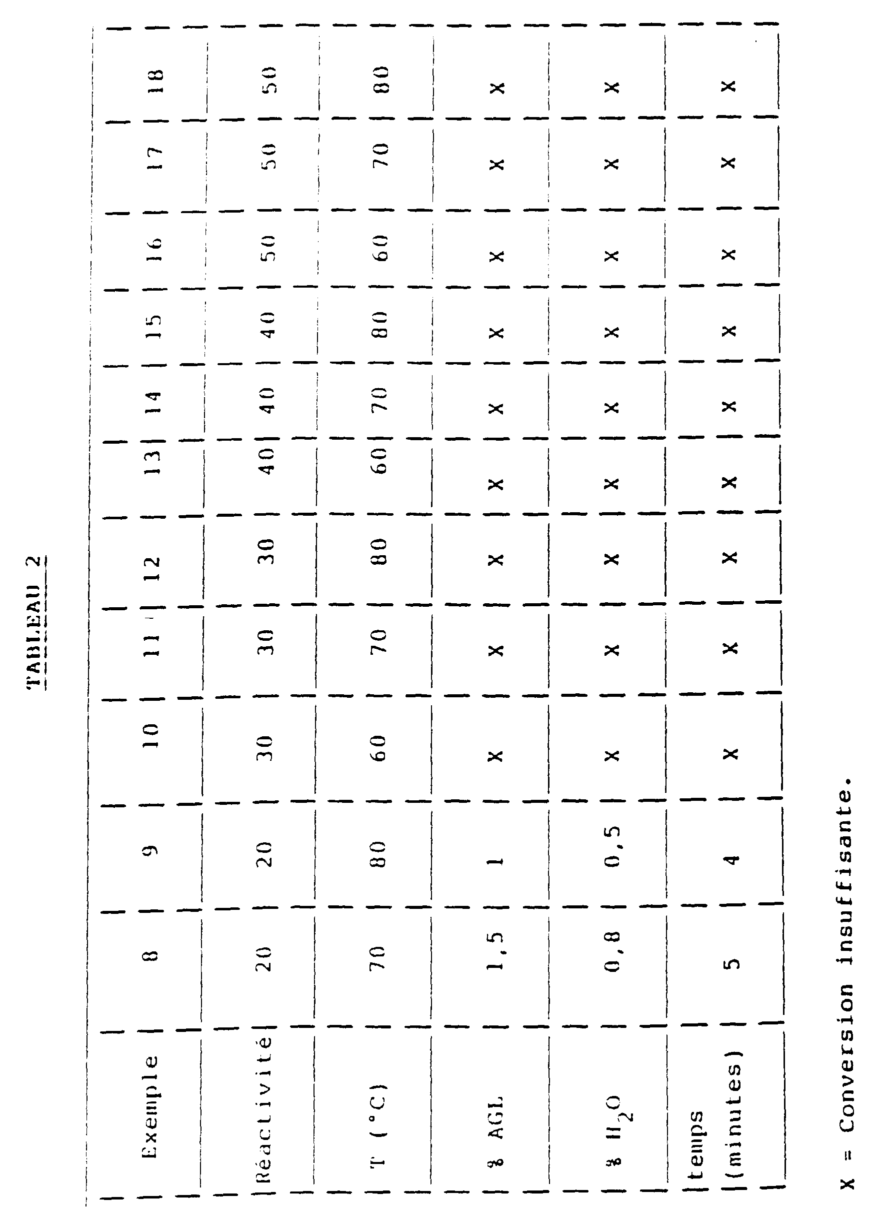

### Exemples 19 à 30

On a répété l'exemple 1 si ce n'est qu'on a maintenu la température de réaction à 70°C, et qu'on a utilisé une quantité différente d'oxyde de magnésium [rapport A = 100 x (oxyde de magnésium/acide stéarique) ; rapport A différent de 10].

Le tableau suivant donne la réactivité de l'oxyde de magnésium utilisé, le rapport (A), la teneur en acides gras libres (% AGL) du stéarate de magnésium, la teneur en humidité (%H₂O) du stéarate de magnésium et le temps de réaction t en secondes.

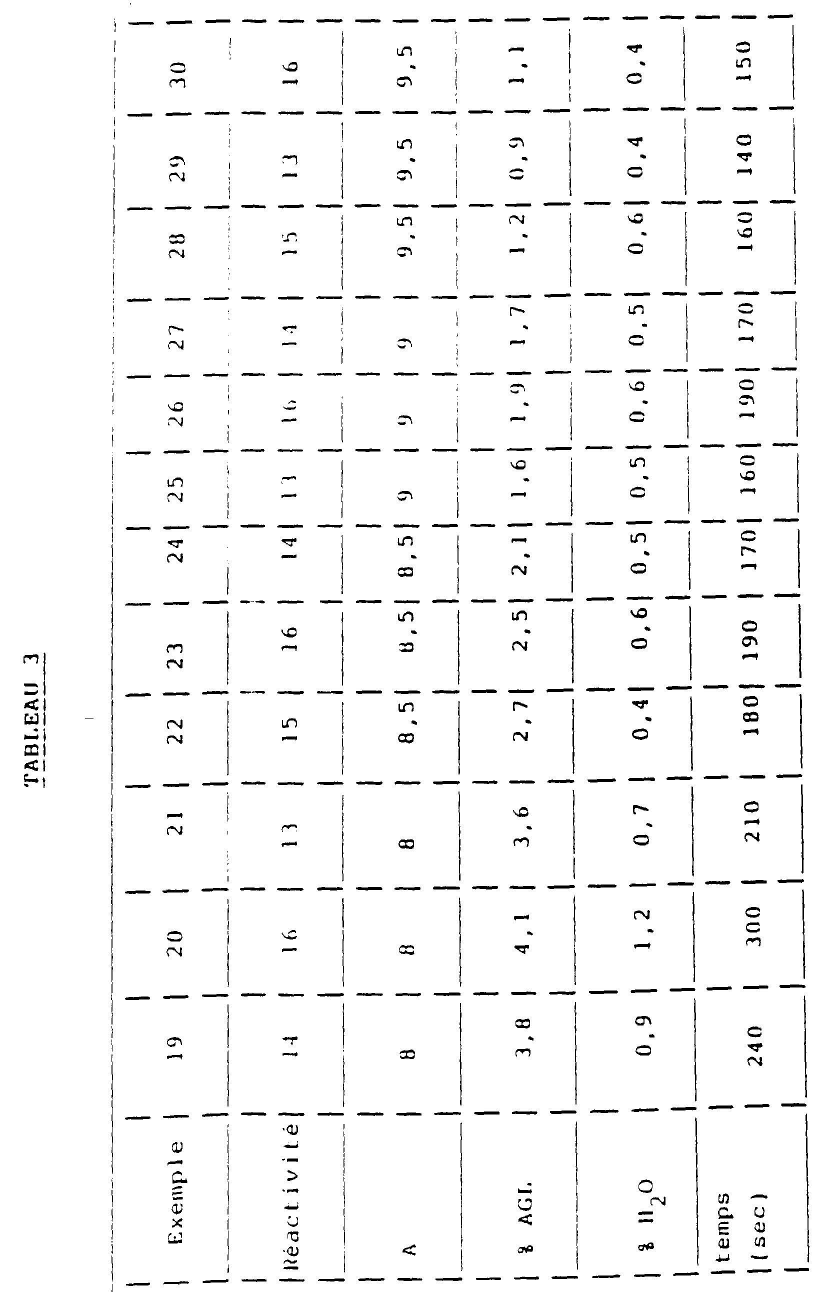

Des exemples 1 à 30, on peut en déduire que des conditions opératoires optimales pour la préparation de stéarate de magnésium selon le procédé suivant l'invention sont :
* 60° C < T < 70°C
* réactivité de l'oxyde de magnésium < 20 s, de préférence comprise entre 14 et 16 s
* (masse de MgO/masse d'acide stéarique) x 100>8,5
* quantité d'eau et/ou d'agent de réactivité est de 7,5 à 8% en masse du mélange initial.

Ces conditions optimales permettent de préparer avec un coefficient d'expansion de la réaction contrôlable et avec un temps de réaction de l'ordre de 3 minutes au stéarate de magnésium qui, en fin de réaction a une teneur en acides gras libres inférieure à 2% et une teneur en humidité inférieure à 0,5%.

### Exemple 31

Dans un premier batch de 1 m³, on a mélangé de l'acide stéarique fondu à 60°C et l'oxyde de magnésium réactif dans les conditions et proportions de l'exemple 1. On dispose, en outre, d'un second batch de 200 l d'eau. Les deux constituants sont pompés simultanément à raison de 500 l/h pour le premier batch et de 35 l/h pour l'eau. Le mélange est réceptionné sur une bande transporteuse ou une installation industrielle similaire, compatible avec le coefficient d'expansion de la réaction et permettant son évolution. La fin de bande est utilisée pour refroidir par ventilation le produit expansé. Le produit de réaction est finalement déversé dans un système de broyage.

Le stéarate de magnésium préparé ainsi en continu présentait les caractéristiques suivantes :
* % AGL < 2
* % H₂O = 0,5

La présente invention a donc pour objet un nouveau procédé. Grâce à des formes d'exécution de ce nouveau procédé, il est possible d'obtenir du stéarate de magnésium présentant une teneur en acides gras libres inférieure à 1 % (voir exemples 8 et 29), voire à 0,5 % (voir exemples 1 à 7).

La présente invention a donc également pour objet un stéarate de magnésium présentant une teneur en acides gras libres inférieure à 1 %, de préférence inférieure à 0,5 %.

## Revendications

1. Procédé de préparation de mono ou dialkanoate, à plus de 5 atomes de carbone, de magnésium dans lequel on fait réagir de l'oxyde et/ou de l'hydroxyde de magnésium avec un ou plusieurs acides carboxyliques possédant plus de 5 atomes de carbone en présence d'eau, caractérisé en ce qu'on prépare dans une première étape un mélange d'acide(s) carboxylique et d'oxyde et/ou d'hydroxyde de magnésium porté à une température telle qu'au moins 80% de l'acide soit sous forme fondue, l'oxyde et/ou l'hydroxyde de magnésium dudit mélange présentant une réactivité inférieure ou égale à 20 secondes, réactivité mesurée par le temps requis pour obtenir le virage au rose de 50 ml d'une solution indicateur portée à 30°C après l'ajout d'1 g d'oxyde et/ou hydroxyde de magnésium, lesdits 50 ml de solution indicateur provenant d'une solution d'acide citrique 0,4 N obtenue en dissolvant 141 g d'acide citrique et 1,25 g de benzoate de sodium à laquelle on a ajouté 10 ml de phénolphtaleine et de l'eau déminéralisée pour obtenir 5000 ml, et en ce qu'on ajoute, dans une deuxième étape, audit mélange de l'eau.

2. Procédé suivant la revendication 1 caractérisé en ce qu'on utilise de l'oxyde de magnésium présentant une réactivité inférieure à 16 secondes.

3. Procédé suivant la revendication 1 ou 2, caractérisé en ce qu'on porte, lors de la première étape, le mélange d'acide(s) carboxylique et d'oxyde et/ou d'hydroxyde de magnésium à une température telle que sensiblement tout l'acide ou les acides carboxylique soient fondus.

4. Procédé suivant l'une quelconque des revendications précédentes, caractérisé en ce que, dans la deuxième étape, on ajoute une quantité d'eau au mélange telle que la teneur en eau du mélange au début de la réaction est d'au moins 3%.

5. Procédé suivant la revendication 4, caractérisé en ce que ladite quantité d'eau ajoutée est telle que la teneur en eau du mélange au début de la réaction est de 5 à 15%.

6. Procédé suivant l'une quelconque des revendications 1 à 5, caractérisé en ce que, dans la première étape, on soumet le mélange à une agitation de manière à avoir un mélange sensiblement homogène.

7. Procédé suivant l'une quelconque des revendications 1 à 6, caractérisé en ce que on fait réagir une quantité d'oxyde et/ou d'hydroxyde de magnésium avec de l'acide ou des acides carboxylique en C₅⁺ telle que le rapport molaire oxyde et/ou hydroxyde de magnésium/acide ou acides carboxyliques en C₅⁺ soit supérieur à 0,5 en début de réaction.

8. Procédé suivant l'une quelconque des revendications 1 à 7, caractérisé en ce qu'on fait réagir de l'oxyde de magnésium et/ou de l'hydroxyde de magnésium avec de l'acide ou des acides carboxyliques en C₅⁺ en présence d'une quantité d'eau telle qu'en fin de réaction, le produit de réaction contient moins de 5% en eau.

9. Procédé suivant l'une quelconque des revendications 1 à 8, caractérisé en ce qu'on fait réagir de l'oxyde de magnésium avec de l'acide stéarique.

10. Procédé suivant l'une quelconque des revendications 1 à 9, caractérisé en ce qu'on ajoute en continu, à un débit de mélange, de l'eau et en ce qu'on laisse réagir le mélange réactionnel de manière à lui permettre une expansion d'au moins 5.

11. Stéarate de magnésium susceptible d'être préparé par le procédé suivant la revendication 1, caractérisé en ce qu'il présente une teneur en acides gras libres inférieure à 1 %.

12. Stéarate de magnésium susceptible d'être préparé par le procédé suivant la revendication 2, caractérisé en ce qu'il présente une teneur en acides gras libres inférieure à 0,5 %.

## Patentansprüche

1. Verfahren zur Herstellung von Magnesiummono- oder -dialkanoat mit mehr als 5 Kohlenstoffatomen, bei welchem man Magnesiumoxid und/oder -hydroxid mit einer oder mehreren, mehr als 5 Kohlenstoffatome aufweisenden Carbonsäuren in Anwesenheit von Wasser zur Reaktion bringt, dadurch gekennzeichnet, daß man in einem ersten Schritt eine Mischung aus Carbonsäure(n) und Magnesiumoxid und/oder -hydroxid zubereitet, die auf eine solche Temperatur gebracht wird, daß sich mindestens 80% der Säure in geschmolzenem Zustand befindet, wobei das Magnesiumoxid und/oder -hydroxid der genannten Mischung eine Reaktivität unter oder gleich 20 s aufweist, welche Reaktivität aus der für die Erzielung des Farbumschlages zu rosa von 50 ml einer auf eine Temperatur von 30°C gebrachten Indikatorlösung erforderlichen Zeit nach Zugabe von 1 g Magnesiumoxid und/oder -hydroxid bestimmt wird, wobei die 50 ml Indikatorlösung aus einer 0,4N Zitronensäurelösung stammen, die hergestellt wurde durch Auflösen von 141 g Zitronensäure und 1,25 g Natriumbenzoat und Zusetzen von 10 ml Phenolphthalein und demineralisiertem Wasser, um 5000 ml zu erhalten, und daß man in einem zweiten Schritt der genannten Mischung Wasser zusetzt.

2. Verfahren nach Anspruch 1, dadurch gekennzeichnet, daß man Magnesiumoxid verwendet, das eine Reaktivität von weniger als 16 s aufweist.

3. Verfahren nach Anspruch 1 oder 2, dadurch gekennzeichnet, daß man während des ersten Schrittes die Mischung aus Carbonsäure(n) und Magnesiumoxid und/oder -hydroxid auf eine solche Temperatur bringt, daß im wesentlichen die gesamte(n) Carbonsäure(n) geschmolzen wird(werden).

4. Verfahren nach irgendeinem der vorhergehenden Ansprüche, dadurch gekennzeichnet, daß man im zweiten Schritt der Mischung eine solche Menge Wasser zusetzt, daß der Wassergehalt der Mischung zu Beginn der Reaktion mindestens 3% beträgt.

5. Verfahren nach Anspruch 4, dadurch gekennzeichnet, daß die zugesetzte Wassermenge so ist, daß der Wassergehalt der Mischung zu Beginn der Reaktion 5 bis 15% beträgt.

6. Verfahren nach irgendeinem der Ansprüche 1 bis 5, dadurch gekennzeichnet, daß man im ersten Schritt die Mischung einem solchen Rühren unterwirft, daß eine im wesentlichen homogene Mischung vorliegt.

7. Verfahren nach irgendeinem der Ansprüche 1 bis 6, dadurch gekennzeichnet, daß man eine solche Menge Magnesiumoxid und/oder -hydroxid mit der oder den mehr als fünf Kohlenstoffatome aufweisenden Carbonsäure(n) zur Reaktion bringt, daß das Molverhältnis von Magnesiumoxid und/oder -hydroxid zu der(den) mehr als fünf Kohlenstoffatome aufweisenden Carbonsäure(n) zu Beginn der Reaktion größer als 0,5 ist.

8. Verfahren nach irgendeinem der Ansprüche 1 bis 7, dadurch gekennzeichnet, daß man das Magnesiumoxid und/oder Magnesiumhydroxid mit der(den) mehr als fünf Kohlenstoffatome aufweisenden Carbonsäure(n) in Gegenwart einer solchen Menge Wasser zur Reaktion bringt, daß am Ende der Reaktion das Reaktionsprodukt weniger als 5% Wasser enthält.

9. Verfahren nach irgendeinem der Ansprüche 1 bis 8, dadurch gekennzeichnet, daß man Magnesiumoxid mit Stearinsäure zur Reaktion bringt.

10. Verfahren nach irgendeinem der Ansprüche 1 bis 9, dadurch gekennzeichnet, daß man einer Fördermenge der Mischung kontinuierlich Wasser zusetzt und daß man das Reaktionsgemisch so reagieren läßt, daß ihm eine Expansion von mindestens 5 erlaubt ist.

11. Magnesiumstearat, herstellbar nach dem Verfahren nach Anspruch 1, dadurch gekennzeichnet, daß es einen Gehalt an freien Fettsäuren von weniger als 1% aufweist.

12. Magnesiumstearat, herstellbar nach dem Verfahren nach Anspruch 2, dadurch gekennzeichnet, daß es einen Gehalt an freien Fettsäuren von weniger als 0,5% aufweist.

## Claims

1. Process for the preparation of magnesium mono or dialkanoate, with more than 5 carbon atoms, in which magnesium oxide and/or hydroxide are made to react with one or several carboxylic acids which have more than 5 carbon atoms in the presence of water, characterised in that in a first step a mixture of carboxylic acid(s) and magnesium oxide and/or hydroxide is prepared and brought to a temperature such that at least 80% of the acid is molten, the magnesium oxide and/or hydroxide of the aforementioned mixture having a reactivity of less than or equal to 20 seconds, reactivity measured by the time required in order to obtain the turning to pink of 50 ml of an indicator solution brought to 30°C after the addition of 1 g of magnesium oxide and/or hydroxide, the aforementioned 50 ml of indicator solution coming from a 0.4 N citric acid solution obtained by dissolving 141 g of citric acid and 1.25 g of sodium benzoate to which is added 10 ml of phenolphthalein and demineralised water in order to obtain 5000 ml, and in that, in a second step, water is added to the aforementioned mixture.

2. Process according to claim 1 characterised in that magnesium oxide with a reactivity of less than 16 seconds is used.

3. Process according to claim 1 or 2, characterised in that during the first step the carboxylic acid(s) and magnesium oxide and/or hydroxide is brought to a temperature such that almost all the carboxylic acid or acids are molten.

4. Process according to any one of the preceding claims, characterised in that, in the second step, a quantity of water is added to the mixture such that the water content of the mixture at the start of the reaction is at least 3%.

5. Process according to claim 4, characterised in that the aforementioned added quantity of water is such that the water content of the mixture at the start of the reaction is 5 to 15%.

6. Process according to any one of the claims 1 to 5, characterised in that, in the first step, the mixture is subjected to an agitation in order to have an almost homogeneous mixture.

7. Process according to any one of the claims 1 to 6, characterised in that a quantity of magnesium oxide and/or hydroxide is made to react with carboxylic acid or acids in C₅⁺ such that the molar ratio of magnesium oxide and/or hydroxide/carboxylic acid or acids in C₅⁺ is greater than 0.5 at the start of the reaction.

8. Process according to any one of the claims 1 to 7, characterised in that magnesium oxide and/or magnesium hydroxide are made to react with carboxylic acid or acids in C₅⁺ in the presence of a quantity of water such that at the end of the reaction, the reaction product contains less than 5% in water.

9. Process according to any one of the claims 1 to 8, characterised in that magnesium oxide is made to react with stearic acid.

10. Process according to any one of the claims 1 to 9, characterised in that water is continuously added to a flow of the mixture and in that the reactive mixture is left to react in order to allow it an expansion of at least 5.

11. Magnesium stearate capable of being prepared with the process according to claim 1, characterised in that it has a free fatty acids content of less than 1%.

12. Magnesium stearate capable of being prepared with the process according to claim 2, characterised in that it has a free fatty acids content of less than 0.5%.
